# EUROPÄISCHE PATENTSCHRIFT

(11) **EP 3 773 459 B1**
(45) Veröffentlichungstag und Bekanntmachung des Hinweises auf die Patenterteilung: **07.06.2023**
(21) Anmeldenummer: 19721817.5
(22) Anmeldetag: 29.03.2019
(51) Int. Cl.: A61K 8/55, A61Q 17/04, A61K 8/02

(54) **VERFAHREN ZUR HERSTELLUNG EINER AUSGANGSFORMULIERUNG FÜR EINE DERMATOLOGISCHE LICHTSCHUTZ-ZUBEREITUNG UND ZUR HERSTELLUNG EINER DERMATOLOGISCHEN LICHTSCHUTZ-ZUBEREITUNG**
PROCESS FOR THE PREPARATION OF A BASE FORMULATION FOR A DERMATOLOGICAL SUNSCREEN COMPOSITION AND FOR THE PREPARATION OF A DERMATOLOGICAL SUNSCREEN COMPOSITION
PROCÉDÉ POUR PRODUIRE UNE FORMULATION DE DÉPART POUR UNE PRÉPARATION DERMATOLOGIQUE DE PROTECTION CONTRE LA LUMIÈRE ET POUR PRODUIRE UNE PRÉPARATION DERMATOLOGIQUE DE PROTECTION CONTRE LA LUMIÈRE

(30) Priorität: 29.03.2018 DE 102018107718
(43) Veröffentlichungstag der Anmeldung: 17.02.2021
(73) Patentinhaber: ULTRASUN AG, 8045 Zürich (CH)
(72) Erfinder: IRNIGER, Benedikt, 8045 Zürich (CH)
(74) Vertreter: Pollard, Peter Julian
(86) Internationale Anmeldenummer: PCT/IB2019/052635
(87) Internationale Veröffentlichungsnummer: WO 2019/186509

(56) Entgegenhaltungen:
- WO-A2-2008/155389
- DE-A1-102006 045 388

## Beschreibung

Die vorliegende Erfindung betrifft ein Verfahren zur Herstellung einer Ausgangsformulierung für eine dermatologische Lichtschutz-Zubereitung, und ein Verfahren zur Herstellung einer dermatologischen Lichtschutz-Zubereitung.

Aus dem Stand der Technik sind diverse dermatologische Lichtschutz-Zubereitungen und deren Herstellungsverfahren bekannt. Siehe WO2008/155389 und DE102006045388.

Lichtschutz-Zubereitungen im Allgemeinen sollen die Haut vor zu viel UV-Strahlung schützen. Am häufigsten findet man Lichtschutz-Zubereitungen in Form von Sonnenmilch. Sonnenmilch ist eine flüssige Emulsion, welche aus einem Fettanteil und einem Wasseranteil besteht. Lichtschutz-Zubereitungen gibt es aber auch in Form von Creme, Öl oder auf Wasser basierenden Gel. Lichtschutz-Zubereitungen mit geringem Wasseranteil sind salbenartig, solche mit hohem Wasseranteil eher wie eine Lotion.

Die üblichen dermatologischen Lichtschutz-Zubereitungen basieren in der Regel auf einer Mischung bekannter und erprobter Kombinationen an Wirk- und Hilfsstoffen. Nicht alle dieser Wirk- und Hilfsstoffe sind gemäss heutiger Erkenntnisse aus medizinischer Sicht und insbesondere auch aus Verbrauchersicht, in Kosmetika und Pflegeprodukten noch erwünscht. Bei vielen dieser Stoffe sind deren Wirkungsweise als Einzelstoff oder als Stoffgemisch, deren Resorption im Körper, deren Freisetzungsgeschwindigkeit, der Ort der Freisetzung und der Ort der Wirkung nämlich oft ungewiss. Dies mag bei Kosmetika bei intakter Haut wegen ihrer hohen Barriere- und Reparaturleistung zwar geduldet werden können, kann aber insbesondere bei sensibler oder geschädigter Haut die beabsichtigte positive Wirkung des Präparats in ihr Gegenteil verkehren.

Einschränkende Regelungen über zulässige Wirk- und Hilfsstoffe finden sich dazu in der europäischen Kosmetikverordnung EG VO 1223/2009.

Bei Lichtschutz-Zubereitungen werden zum Blockieren der UV-Strahlung, als Wirkstoffe üblicherweise chemische und physikalische Filterstoffe eingesetzt.

Chemische Filter, beispielsweise in einer Zubereitung wie in der DE 693 189 12 T2 beschrieben, sind zwar überaus effizient, da ihre Wirkung durch das Absorptionsverhalten der Haut besonders lange anhalten kann. Sie stehen jedoch im Verdacht, möglicherweise allergische Reaktionen oder Zellschädigungen auszulösen.

Physikalische Filter bestehen aus sehr fein gemahlenen Mineralstoffen, vorzugsweise Metalloxiden wie beispielsweise Titandioxid, Zinkoxid oder Aluminiumoxid und die Filterwirkung ist rein physikalischer Natur durch Streuung, Reflektion und teilweiser Absorption mit einhergehender Energieumwandlung des UV-Lichts. Ihr Vorteil ist, dass sie chemisch inert sind und sich deshalb weder zersetzen noch allergische Nebenwirkungen auslösen (mit Ausnahme von Aluminiumoxid, weshalb dies im Sinne der Erfindung ausgenommen wird). Ein Nachteil ist jedoch, dass die Schutzschicht aus der aufgetragenen Lichtschutz-Zubereitung durch Wasser oder Schweiss leicht abgespült werden kann. Ungewollt und meistens unbemerkt kann es daher zu einer Hautschädigung durch UV-Licht kommen, weshalb zum Nachteil des Anwenders öfter nachgecremt werden muss.

Zur Steigerung des Anwendungskomforts sowie der Hautverträglichkeit von Lichtschutz-Zubereitungen ist es aus den oben genannten Gründen wünschenswert, dass Lichtschutz-Zubereitungen sowohl überwiegend physikalische Filter enthalten als auch lang anhaltenden Schutz bieten und darüber hinaus wasserfest sind. Des Weiteren sollen Lichtschutz-Zubereitungen effizient und wirtschaftlich hergestellt werden können.

Wünschenswert wäre daher ein einfach handhabbares Herstellungsverfahren für eine dermatologische Lichtschutz-Zubereitung, bei welcher der Anteil unerwünschter Stoffe im Verhältnis zu den unbedenklichen Hilfs- und Wirkstoffen deutlich verringert, wenn nicht gar vollständig ausgeschlossen ist, um so unerwünschte Wirkungen zu minimieren.

Als besonders vielversprechende Lichtschutz-Zubereitungen haben sich dabei Zubereitungen auf der Basis lamellarer Schichtsysteme, vorzugsweise sogenannte "hydrierter Liposomen", bewährt. Solche lamellaren Schichtsysteme und deren Herstellungsverfahren sind beispielsweise für kosmetische Gesichtspflege- und Lippenpflege in der DE 10 2006 045 388 A1 oder auch in der DE 10 2006 045 389 A1 offenbart,

Bekannte Herstellungsverfahren haben sich jedoch als nicht optimal praktikabel erwiesen. Derart hergestellte Lichtschutz-Zubereitungen mit einer liposomalen Phase und in dieser integrierte chemische und physikalische Filter, insbesondere beschrieben in der DE 10 2006 045 388 A1, sind nicht stabil, es kommt zur Phasentrennung. Ebenso ist das Herstellungsverfahren sehr aufwendig, nicht ohne Weiteres skalierbar und damit eben auch nicht effizient und einfach handhabbar.

Es ist daher Aufgabe der Erfindung, ein Verfahren zur Herstellung einer Ausgangsformulierung für eine dermatologische Lichtschutz-Zubereitung zur Verfügung zu stellen, welches die zuvor genannten Nachteile aus dem Stand der Technik überwindet.

Im Sinne der Erfindung ist die Ausgangsformulierung eine Ausgangsformulierung, welche eine Ölphase und eine Wasserphase umfasst, und wenigstens eine UV-absorbierende Substanz sowie wenigstens eine amphiphile Substanz enthält, die lamellaren Strukturen, vorzugsweise ein System aus lamellaren Strukturen, ausbildet. Lamellare Strukturen bilden Systeme aus, welche sich durch einen regelmässigen, schichtartigen Aufbau auszeichnen. Im menschlichen Körper finden sich solche Strukturen als Lipiddoppelschichten in den Zellmembranen.

Topische lamellare Systeme orientieren sich in der Regel an den natürlichen Vorbildern und nutzen Phosphatidylcholin, Ceramide, Sterine und Fettsäuren als strukturbildende Elemente.

Im Sinne der Erfindung können lamellare Systeme jedoch auch von vielen synthetischen Tensiden gebildet werden, wenn sie in ausreichend hoher Konzentration vorliegen und diese amphiphilen Substanzen aufgrund ihrer molekularen Struktur, das heisst einem ausgewogenen Verhältnis von hydrophilem und lipophilem Molekülteil, eine Doppelschichtanordnung zulassen.

Die Ausbildung lamellarer Strukturen hydrierter Lipide ist jedoch nicht spontan, sondern erfordert bei der Herstellung, insbesondere bei der Barrierekonstitution, einen hohen Energieeintrag in Form von Temperatur und gegebenenfalls mechanische Energie (zum Beispiel Homogenisieren unter Druck und/oder Rühren).

Im Sinne der Erfindung erzeugt man für die Herstellung einer Ausgangsformulierung für eine dermatologische Lichtschutz-Zubereitung eine Ölphase und eine Wasserphase, wie in Anspruch 1 definiert, die man vereinigt und dann homogenisiert.

Bei bevorzugten Verfahren enthält die Ausgangsformulierung eine Ölphase mit einem Anteil von 30 bis 50 Gewichtsprozent, bevorzugt 25 bis 40 Gewichtsprozent, oder auch 35 bis 45 Gewichtsprozent und eine Wasserphase mit einem Anteil von 30 bis 40 Gewichtsprozent, bevorzugt 25 bis 40 Gewichtsprozent, oder auch 35 bis 45 Gewichtsprozent.

Bei einer bevorzugten Ausführungsform eines erfindungsgemässen Verfahrens enthält die Ausgangsformulierung eine Ölphase und eine Wasserphase in einem Verhältnis von 1:4 bis 4:1, vorzugsweise von 1:3 bis 3:1 und besonders bevorzugt von 1:2 bis 2:1.

Bei einer bevorzugten Ausführungsform eines erfindungsgemässen Verfahrens enthält die Ausgangsformulierung mehrheitlich, vorzugsweise nahezu, und für spezielle Anwendungen ausschliesslich, eine Ölphase sowie einen geringen Anteil von unter 10 Gewichtsprozent, vorzugsweise unter 5 Gewichtsprozent, an einer Wasserphase.

Bei bevorzugten Verfahren enthält die Ölphase wenigstens ein, vorzugsweise zwei, mittelkettige Triglyceride, ausgewählt aus Veresterungsprodukten von Glycerin mit Caprinsäure und Caprylsäure im Verhältnis 1:3, vorzugsweise Capryl-Triglyceride, und Coco-Triglyceride.

Bei erfindungsgemässen Verfahren enthält die Ölphase als UV-Absorber ein Hexyl Benzoat, vorzugsweise ein 2-[4-(Diethylamino)-2-hydroxybenzoyl]-benzoesäurehexylester und/oder ein Bemotrizinol, und/oder ein Cyanurtriamid und/oder ein Ethylhexyltriazon, und/oder ein Ethylhexylsalicylat und/oder ein, vorzugsweise beschichtetes, TiO₂.

Im Sinne der Erfindung ist beschichtetes TiO₂ ein TiO₂ Pulver, dessen Partikel mit einer Silikatschicht überzogen wurden. Dies hat zum Vorteil, dass durch die Beschichtung die UV-Absorbtionsrate gegenüber unbeschichteten TiO₂ höher ist und damit ein grösserer Schutz gegenüber UV-Strahlung bei gleichbleibender Menge TiO2 gegeben ist.

Bei bevorzugten Verfahren enthält die Ölphase einen Anteil Phytosqualan von 0,5 bis 6,5, vorzugsweise bis 8,5 oder auch bis 10,5 Gewichtsprozent.

Bei bevorzugten Verfahren enthält die Ölphase ein Anteil Alkyl-Benzoat von 3,5 bis 9,5, vorzugsweise bis 14,5 oder auch bis 19,5 Gewichtsprozent.

Bei bevorzugten Verfahren enthält die Ölphase einen Anteil, vorzugsweise beschichtetes, TiO₂ von 6,5 bis 15,5, vorzugsweise bis 18,5 oder auch bis 20,5 Gewichtsprozent als physikalischen Filterstoff. In Versuchen hat sich dabei ergeben, dass sich, vorzugsweise beschichtetes, TiO₂ in Form von nanokristallinem Pulver mit einer durchschnittlichen Korngrösse von kleiner 30 nm bis 25 nm, vorzugsweise kleiner 25 nm bis 20 nm, bevorzugt kleiner 20 nm bis 15 nm oder kleiner 15 nm bis 10 nm und besonders bevorzugt kleiner 10 nm bis 5 nm besonders eignet.

Bei bevorzugten Verfahren stehen die UV-absorbierende Substanzen in der Ölphase zu der UV-absorbierenden anorganischen Substanz in einem Verhältnis von wenigstens 1:1, vorzugsweise wenigstens 1:2 bis 2:1 oder auch wenigstens 1:1,5 bis 1,5:1, besonders bevorzugt wenigstens 1:3 bis 3:1, und höchstens 1:4 bis 4:1.

Bei bevorzugten Verfahren enthält die Wasserphase eine amphiphile Substanz, vorzugsweise hydriertes Phosphatidylcholin mit 0,5 bis 5,5, vorzugsweise bis zu 10,5 oder auch bis zu 12,5 Gewichtsprozent.

Hydriertes Phosphatidylcholin weisst eine besonders geeignete Fettsäurebesetzung, bestehend aus gesättigten C₁₈- und C₁₆-Säuren, auf, welche den planaren Aufbau einer Doppelschicht bestimmen.

Bei bevorzugten Verfahren wird vorzugsweise mittels

Säulenchromatographie aus Soja-Lecithin fraktioniertes natives Phosphatidylcholin mit einem hohen Linolsäuregehalt von 80 bis 90 Gewichtsprozent in der Fettsäurebesetzung verwendet, welches zelluläre Doppelschichten erzeugt. Solche Doppelschichten, auch als Liposome bekannt, verstärken die Penetration von Wirkstoffen, insbesondere physikalischer Filter.

Bei bevorzugten Verfahren können somit durch Einstellung geeigneter Mischungsverhältnis aus Ölphase und/oder Wasserphase und/oder der darin umfassten UV-absorbierender und/oder UV-absorbierender anorganischer Substanzen und/oder amphiphiler Substanzen Ausgangsformulierung mit einem hohen Transportgrad an Wirkstoffen und einem sehr geringen Auswascheffekt aus der Haut stufenlos eingestellt werden.

Bei erfindungsgemässen Verfahren enthält die Wasserphase Glycerin und/oder Glykol, vorzugsweise Pentylenglykol.

Bei bevorzugten erfindungsgemässen Verfahren wird der Wasserphase wenigsten ein, vorzugsweise anionisches gemischtes, Polysaccharid, zugegeben.

Im Sinne der Erfindung dient die Zugabe von wenigstens einem Gelbildner, vorzugsweise das zuvor genannte Polysaccharid oder ein Carbomer, der Stabilisierung der lamellaren Strukturen bzw. dem lamellaren System gegenüber Konsistenzänderungen oder Lysophosphatidylcholin-Bildung (Abspaltung von Fettsäureresten durch Hydrolyse).

Bei dem beanspruchten Verfahren wird die Ölphase in dem ersten Schritt auf eine maximale Temperatur von 83 °C, und in dem zweiten Schritt auf eine maximale Temperatur von 76 °C aufgeheizt. Bei bevorzugten Verfahren ist die weitere UV-absorbierende Substanz ein, vorzugsweise auf Bisoctrizol basierender, UV-A/UV-B Absorber.

Bei einem erfindungsgemässen Verfahren zur Herstellung einer dermatologischen Lichtschutz-Formulierung, erzeugt man eine zweite Wasserphase, die neben Wasser wenigstens einen Polyalkohol enthält; und vermischt die erfindungsgemässe Ausgangsformulierung mit der zweiten Wasserphase bei einer Temperatur von wenigstens 60 °C und homogenisiert das Gemisch.

Bevorzugt umfasst der wenigstens eine Polyalkohol der zweiten Wasserphase ein Glycerin und/oder ein Glykol, vorzugsweise Pentylenglykol.

## Patentansprüche

1. Verfahren zur Herstellung einer Ausgangsformulierung für eine dermatologische Lichtschutz-Zubereitung,
wobei die Ausgangsformulierung eine Ölphase und eine Wasserphase umfasst und mehrere UV-absorbierende Substanzen sowie wenigstens eine amphiphile Substanz enthält, die lamellare Strukturen ausbildet, bei dem man eine Ölphase erzeugt, die wenigstens ein Triglycerid und den wesentlichen Gehalt an UV-absorbierenden Substanzen umfasst, und diese Ölphase in einem ersten Schritt auf wenigstens 80°C und maximal 83°C erhitzt und homogenisiert und in einem zweiten Schritt bei wenigstens 70°C und maximal 76°C wenigstens eine UV-absorbierende anorganische Substanz beimischt; eine Wasserphase erzeugt, die den wesentlichen Gehalt an amphiphiler Substanz umfasst, und diese Wasserphase auf wenigstens 70°C erhitzt und homogenisiert;
beide Phasen, ggf. unter Zugabe wenigstens einer weiteren UV-absorbierenden Substanz, vereinigt und das vereinigte Produkt homogenisiert.

2. Verfahren nach Anspruch 1, wobei die Ölphase wenigstens ein, vorzugsweise zwei, mittelkettige Triglyceride enthält, ausgewählt aus Capryl-Triglyceriden und Coco-Triglyceriden.

3. Verfahren nach einem der vorherigen Ansprüche, wobei die Ölphase als UV-Absorber
a. ein Hexyl-Benzoat, und/oder
b. ein Bemotrizinol, und/oder
c. ein Ethylhexyltriazon, und/oder
d. ein Ethylhexylsalicylat, und/oder
e. ein Ti02, vorzugsweise beschichtet,
enthält.

4. Verfahren nach einem der vorherigen Ansprüche, wobei die Ölphase einen Anteil Phytosqualan von 1 bis 8,5 Gewichtsprozent enthält.

5. Verfahren nach einem der vorherigen Ansprüche, wobei die Ölphase ein Anteil Alkyl-Benzoat von 3,5 bis 19,5 Gewichtsprozent enthält.

6. Verfahren nach einem der vorherigen Ansprüche, wobei die Ölphase ein Anteil Ti02 von 7 bis 15,5 oder auch bis 20,5 Gewichtsprozent enthält.

7. Verfahren nach einem der vorherigen Ansprüche, wobei die UV-absorbierende Substanzen in der Ölphase zu der UV-absorbierenden anorganischen Substanz in einem Verhältnis von wenigstens 1:1, vorzugsweise wenigstens 1:2 bis 2:1 oder auch wenigstens 1:1,5 bis 1,5:1, besonders bevorzugt wenigstens 1:3 bis 3:1, und höchstens1:4 bis 4:1 stehen.

8. Verfahren nach einem der vorherigen Ansprüche, wobei die Wasserphase eine amphiphile Substanz, vorzugsweise hydriertes Phosphatidylcholin, mit 0,5 bis 10,5 Gewichtsprozent enthält.

9. Verfahren nach einem der vorherigen Ansprüche, wobei die Wasserphase Glycerin und/oder Glykol, vorzugsweise Pentylenglykol enthält.

10. Verfahren nach einem der vorherigen Ansprüche, wobei die weitere UV-absorbierende Substanz, vorzugsweise ein auf Bisoctrizol basierender UV-A/UV-B Absorber ist.

11. Verfahren zur Herstellung einer dermatologischen Lichtschutz-Formulierung, bei dem man eine zweite Wasserphase erzeugt, die neben Wasser wenigstens einen Polyalkohol enthält;
und die Ausgangsformulierung gemäss Anspruch 1 mit der zweiten Wasserphase bei einer Temperatur von wenigstens 60°C vermischt und das Gemisch homogenisiert.

12. Verfahren nach Anspruch 11, wobei der wenigstens eine Polyalkohol der zweiten Wasserphase ein Glycerin und/oder Glykol, vorzugsweise Pentylenglykol, umfasst.

## Claims

1. Process for the preparation of a base formulation for a dermatological sunscreen composition, wherein the base formulation comprises an oil phase and a water phase and contains a plurality of UV-absorbing substances and at least one amphiphilic substance which forms lamellar structures, at which an oil phase is produced which comprises at least one triglyceride and the substantial content of UV-absorbing substances, and said oil phase is heated and homogenized in a first step to at least 80°C and at most 83°C, and at least one UV-absorbing inorganic substance is admixed in a second step at at least 70°C and at most 76°C; a water phase is produced which comprises the substantial content of amphiphilic substance, and said water phase is heated to at least 70°C and homogenised; both phases are combined, optionally with the addition of at least one further UV-absorbing substance, and the combined product is homogenised.

2. Process according to claim 1, wherein the oil phase contains at least one, preferably two, medium-chain triglycerides, chosen from caprylic triglycerides and coco triglycerides.

3. Process according to any one of the preceding claims, wherein the oil phase contains as UV absorber
a. a hexyl benzoate, and/or
b. a bemotrizinol, and/or
c. an ethylhexyl triazone, and/or
d. an ethylhexyl salicylate, and/or
e. a TiO2, preferably coated.

4. Process according to any one of the preceding claims, wherein the oil phase contains a proportion of phytosqualane of from 1 to 8.5% by weight.

5. Process according to any one of the preceding claims, wherein the oil phase contains a proportion of alkyl benzoate of from 3.5 to 19.5% by weight.

6. Process according to any one of the preceding claims, wherein the oil phase contains a proportion of TiO2 of from 7 to 15.5 or also up to 20.5 % by weight.

7. Process according to any one of the preceding claims, wherein the UV-absorbing substances in the oil phase are in a ratio of at least 1:1, preferably at least 1:2 to 2:1 or also at least 1:1.5 to 1.5:1, particularly preferably at least 1:3 to 3:1, and at most1:4 to 4:1, to the UV-absorbing inorganic substance.

8. Process according to any one of the preceding claims, wherein the water phase contains an amphiphilic substance, preferably hydrogenated phosphatidylcholine, with 0.5 to 10.5% by weight.

9. Process according to any one of the preceding claims, wherein the water phase contains glycerol and/or glycol, preferably pentylene glycol.

10. Process according to any one of the preceding claims, wherein the further UV absorbing substance is preferably a bisoctrizole-based UV-A/UV-B absorber.

11. Process for the preparation of a dermatological sunscreen formulation, in which a second water phase is produced which, in addition to water, contains at least one polyalcohol;
and the base formulation according to claim 1 is mixed with the second water phase at a temperature of at least 60°C and the mixture is homogenised.

12. Process according to claim 11, wherein the at least one polyalcohol of the second water phase comprises a glycerol and/or glycol, preferably pentylene glycol.

## Revendications

1. Procédé de fabrication d'une formulation de base pour une préparation dermatologique de protection contre la lumière, ladite formulation de base comprenant une phase huileuse et une phase aqueuse et contenant plusieurs substances absorbant les UV ainsi qu'au moins une substance amphiphile formant des structures lamellaires, dans lequel on prépare une phase huileuse comprenant au moins un triglycéride et l'essentiel des substances absorbant les UV et chauffe cette phase huileuse dans une première étape à au moins 80°C et au maximum 83°C et on l'homogénéise et y mélange dans une deuxième étape à au moins 70°C et au maximum 76°C au moins une substance inorganique absorbant les UV; produit une phase aqueuse comprenant le contenu substantiel de la substance amphiphile, et chauffe cette phase aqueuse à au moins 70°C et l'homogénéise;
combine les deux phases, éventuellement en ajoutant au moins une autre substance absorbant les UV, et homogénéiser le produit combiné.

2. Procédé selon la revendication 1, dans lequel la phase huileuse contient au moins un, de préférence deux, triglycérides à chaîne moyenne choisis parmi les triglycérides capryliques et les triglycérides coco.

3. Procédé selon l'une quelconque des revendications précédentes, dans lequel la phase huileuse contient, en tant qu'absorbeur d'UV, un ou plusieurs des éléments suivants
a. un benzoate d'hexyle, et/ou
b. un bemotrizinol, et/ou
c. une éthylhexyltriazone, et/ou
d. un salicylate d'éthylhexyle, et/ou
e. un TiO2, de préférence enrobé.

4. Procédé selon l'une quelconque des revendications précédentes, dans lequel la phase huileuse contient une proportion de phytosqualane comprise entre 1 et 8,5 pourcent en poids.

5. Procédé selon l'une quelconque des revendications précédentes, dans lequel la phase huileuse contient une proportion de benzoate d'alkyle comprise entre 3,5 et 19,5 pourcent en poids.

6. Procédé selon l'une quelconque des revendications précédentes, dans lequel la phase huileuse contient une teneur en TiO2 de 7 à 15,5, voire jusqu'à 20,5 pourcent en poids.

7. Procédé selon l'une quelconque des revendications précédentes, dans lequel les substances absorbant les UV dans la phase huileuse sont dans une proportion d'au moins 1:1, de préférence d'au moins 1:2 à 2:1, ou encore d'au moins 1:1,5 à 1,5:1, de manière particulièrement préférée d'au moins 1:3 à 3:1, et d'au plus 1:4 à 4:1.

8. Procédé selon l'une quelconque des revendications précédentes, dans lequel la phase aqueuse contient une substance amphiphile, de préférence de la phosphatidylcholine hydrogénée, à raison de 0,5 à 10,5 pourcent en poids.

9. Procédé selon l'une quelconque des revendications précédentes, dans lequel la phase aqueuse contient du glycérol et/ou du glycol, de préférence du pentylène glycol.

10. Procédé selon l'une quelconque des revendications précédentes, dans lequel l'autre substance absorbant les UV est, de préférence, un absorbant UV-A/UV-B à base de bisoctrizole.

11. Procédé de préparation d'une formulation dermatologique de protection contre la lumière, dans lequel on prépare une seconde phase aqueuse contenant, outre de l'eau, au moins un polyalcool ; et on mélange la formulation de base de la revendication 1 avec la deuxième phase aqueuse à une température d'au moins 60°C et on homogénéise le mélange.

12. Procédé selon la revendication 11, dans lequel ledit au moins un polyalcool de la deuxième phase aqueuse comprenant un glycérol et/ou un glycol, préférablement du pentylène glycol.
